# EUROPÄISCHE PATENTSCHRIFT

(11) **EP 3 658 105 B1**
(45) Veröffentlichungstag und Bekanntmachung des Hinweises auf die Patenterteilung: **14.01.2026**
(21) Anmeldenummer: 18729358.4
(22) Anmeldetag: 24.05.2018
(51) Int. Cl.: A61K 8/06, A61K 8/73, A61K 8/894, A61Q 19/00

(54) **WASSER-IN-ÖL-EMULSIONEN AUF DER GRUNDLAGE VON CETYL DIGLYCERYL TRIS (TRIMETHYLSILOXY)-SILYLETHYLDIMETHICONE ALS EMULGATOR, WELCHE EINEN GEHALT AN EINER ODER MEHREREN PARTIKULÄREN UND/ODER NICHT-WASSERQUELLBAREN STÄRKEN UND/ODER STÄRKEDERIVATEN AUFWEISEN**
WATER-IN-OIL EMULSIONS WHICH ARE BASED ON CETYL DIGLYCERYL TRIS(TRIMETHYLSILOXY)SILYL ETHYL DIMETHICONES AS EMULSIFIER AND CONTAIN ONE OR MORE PARTICULATE AND/OR NON-WATER-SWELLABLE STARCHES AND/OR STARCH DERIVATIVES
ÉMULSIONS EAU DANS L'HUILE CONTENANT DES CÉTYL-DIGLYCÉRYL-TRIS-(TRIMÉTHYLSILOXY)-SILYLÉTHYLDIMÉTHICONES COMME ÉMULSIFIANT ET CONTENANT UN OU PLUSIEURS AMIDONS ET/OU DÉRIVÉS D'AMIDON PARTICULAIRES ET/OU NE POUVANT PAS GONFLER DANS L'EAU

(30) Priorität: 24.07.2017 DE 102017212626
(43) Veröffentlichungstag der Anmeldung: 03.06.2020
(73) Patentinhaber: Beiersdorf AG, 22529 Hamburg (DE)
(72) Erfinder: CHANTY, Delphine, 20253 Hamburg (DE); RASCHKE, Thomas, 25421 Pinneberg (DE)
(74) Vertreter: Beiersdorf AG
(86) Internationale Anmeldenummer: PCT/EP2018/063651
(87) Internationale Veröffentlichungsnummer: WO 2019/020242

(56) Entgegenhaltungen:
- WO-A1-2018/133995
- US-A1- 2001 012 860
- US-A1- 2015 011 656
- US-A1- 2016 038 397
- GUTKOWSKI SARAH ET AL: "Water-in-oil and oil-in-water emulsions stabilized by octenylsuccinic anhydride modified starch and adsorption of modified starch at emulsified oil/water interfaces", 1 January 2016 (2016-01-01), pages 1 - 55, XP055920020, Retrieved from the Internet <URL:https://krex.k-state.edu/dspace/bitstream/handle/2097/32842/SarahGutkowski2016.pdf?sequence=1&isAllowed=y> [retrieved on 20220511]
- CINDY DELVALL? ET AL: "New Formulation Possibilities with a Water-in-oil Silicone Emulsifier Suitable for PEG-free Systems", 31 December 2014 (2014-12-31), XP055349098, Retrieved from the Internet <URL:http://www.dowcorning.com/content/publishedlit/27-1463-water-in-oil-silicone-emulsifier.pdf> [retrieved on 20170223]

## Beschreibung

Die vorliegende Erfindung betrifft eine kosmetische Zubereitung Zubereitungen in Form von Wasser-in-Öl-Emulsionen auf der Grundlage von Cetyl Diglyceryl Tris-(Trimethylsiloxy)silyl-ethyldimethicone als Emulgator, welche einen Gehalt an einer oder mehreren partikulären und/oder nicht-wasserquellbaren Stärken und/oder Stärkederivaten, gewählt aus der Gruppe Tapiocastärke, aufweisen.

Anwender stellen an moderne Hautpflegeprodukte, speziell in Form von Cremes für die Anwendung im Gesicht, eine Reihe von Anforderungen. Sie sollen die Haut pflegen, insbesondere die Haut über einen langen Zeitraum mit Feuchtigkeit versorgen. Dieses wird in der Regel über "Moisturizer" realisiert, die Wasser im Stratum Corneum binden (z.B. Glycerin), zudem werden mit zugesetzten Lipiden die oberen Hautschuppen geschmeidig gehalten und der transepidermale Wasserverlust reduziert.

Des Weiteren erwarten die Anwender häufig eine Wirkung gegen die Anzeichen von Hautalterung, wie z.B. die Ausbildung von Falten, das Nachlassen der Hautelastizität oder die Entstehung von Altersflecken. Hierzu werden zum einen Wirkstoffe verwendet, die beispielsweise die Aktivität der Hautzellen stimulieren, aber auch verstärkt UV-Filter eingesetzt, die besonders für die Hautalterung verantwortlichen UVA-Strahlen blockieren. Alle diese Inhaltsstoffe stellen eine große Herausforderung für eine angenehme Sensorik dar, da sie nach dem Verdunsten der flüchtigen Bestandteile der Formulierung als Film auf der Haut zurückbleiben, und dort sensorisch vom Verbraucher wahrgenommen werden. Die fühlbaren Rückstände werden dabei oft negativ beschrieben und verhindern das Gefühl, dass das Produkt komplett in die Haut "eingezogen" sei.

Konventionelle W/O Emulsionen werden gerne von Verbrauchern verwendet, wenn sie eine trockene Haut pflegen oder behandeln wollen. Vorteile bieten W/O Emulsionen auch bei der Einarbeitung von Farbpigmenten, z.B. in getönten Tagescremes und Make-up. Leider zeichnen sich viele W/O Emulsionen aber dadurch negativ aus, dass sie einen öligen Rückstand auf der Haut hinterlassen, der nur langsam einzieht. Darüber hinaus ist ihre physikalische Stabilität oft begrenzt. Das ist besonders dann problematisch, wenn W/O Emulsionen mit größeren Mengen an mikropartikulären Puderrohstoffen bzw Sensorikadditiven formuliert werden sollen. Typische Beispiele für diese mikropartikulären Sensorikadditive sind Aluminum Starch Octenylsuccinat und Nylon-12.

Silikonbasierte Emulgatoren haben fast ausnahmslos PEG- und PPG-haltige Gruppen als hydrophile Komponente. Typische Vertreter sind Cetyl PEG/PPG-10/1 Dimethicone (Abil EM 90, 180) von Evonik oder PEG-10 Dimethicone (ES-5612 Formulation Aid) von Dow Corning. Diese Beispiele sind erfolgreiche und seit langem etablierte W/O Emulgatoren im Markt. Die Auswahl an PEG-freien Silikonemulgatoren ist aber beschränkt. Man findet hier beispielsweise KF-6100, KF-6104, KF-6105 oder KSG-710, KSG-810, KSG-820, KSG-830 oder KSG-840 von Shin Etsu oder Abil EM 120 von Evonik.

Üblicherweise werden diese W/Si Silikonemulgatoren in hohen Konzentrationen von mehreren Prozent eingesetzt. Die Auswahl der Öle für W/O Emulsionen sowie insbesondere für W/Si Emulsionen ist begrenzt, da für eine gute Langzeitstabilität ein hoher Anteil an unpolaren Ölen erforderlich ist. Daher kommen in W/Si Emulsionen im Wesentlichen Silikonöle und / oder flüssige Kohlenwasserstoffe zum Einsatz.

Aufgabe der vorliegenden Erfindung ist daher, eine stabile (Lagerstabile, transportstabile, langzeitstabile) Wasser-in-Öl- (W/O-) Emulsion zur Verfügung zu stellen, die sensorisch attraktiv, PEG-frei und in die Stärke als mikropartikuläres Sensorikadditiv eingearbeitet werden können, dabei aber alle Anforderungen an ein modernes Gesichtspflege-Produkt erfüllt.

Insgesamt bleibt das Hauptproblem, dass Wasser-in-Öl-Emulsionen zu Instabilität neigen. Bekannte Übelstände dieser Art sind Ölabscheidung und Phasentrennung. Das Problem wird noch größer, wenn mikro-partikuläre Rohstoffe eingebaut werden müssen. Zwar kann dem durch Verwendung bekannter Stabilisatoren (beispielsweise Hektorite) entgegengewirkt werden, oder der Anteil der Ölphase kann vermindert werden. Dadurch müssen dann aber wiederum andere Nachteile in Kauf genommen werden, beispielsweise, dass sich polare lipophile Substanzen wie beispielsweise UV-Filter schlechter einarbeiten lassen.

Aufgabe der vorliegenden Erfindung war es daher, diesen Übelständen entgegenzuwirken. Ein besonders interessanter Emulgator wird von Dow Corning unter der INCI- Bezeichnung Cetyl Diglyceryl Tris-(Trimethylsiloxy)silylethyldimethicone und unter der Handelsbezeichung "ES-5600" verkauft.

WO2018133995 A1 offenbart in Beispiel 9 eine W/O-Emulsion enthaltend A) 1.5% Cetyl Diglyceryl Tris-(Trimethylsiloxy)silylethyldimethicone (ES-5600), B) Cetearylisononanoate und Dimethicone als Ölphase, C) Wasser und Tapiocastärke, und ist demnach vom Patentschutz ausgenommen.

Es war daher überraschend und für den Fachmann nicht vorhersehbar, dass die der Erfindung zugrundeliegenden Aufgaben gelöst werden durch kosmetische oder dermatologische Wasser-in-Öl Emulsionen, umfassend
A) Cetyl Diglyceryl Tris-(Trimethylsiloxy)silylethyldimethicone als Emulgator
B) eine Ölphase
C) eine wässrige Phase,
gekennzeichnet durch einen Gehalt an einer oder mehreren partikulären und/oder nicht-wasserquellbaren Stärken und/oder Stärkederivaten, gewählt aus der Gruppe Tapiocastärke.

Stärke gehört zu den Homoglycanen. Sie ist ein Polykondensationsprodukt von d-Glucose. Sie besteht aus 3 verschiedenen d-Glucopyranose-Polymeren, der Amylose, dem Amylopektin und einer sogenannten Zwischenfraktion, die auch als anormales Amylopektin bezeichnet wird. Stärke kann vernetzt und unvernetzt vorliegen. Distärkephosphat ist ein solches Vernetzungsprodukt, bei welcher die Phosphat-Gruppe eine Brücke zwischen zwei Stärkeketten bildet. Die Herstellung erfolgt durch Umsetzung von nativer Stärke in wässriger Suspension mit Natriumtrimetaphosphat, Phosporoxychlorid oder Phosphorpentachlorid.

Vorteilhafte Stärken oder Stärkederivate werden gewählt aus der Gruppe: Maisstärke Zea Mays (Amidon De Mais MST (Wackherr), Argo Brand Corn Starch (Corn Products), Pure-Dent (Grain Processing), Purity 21C (National Starch), Farmal CS 3400 (Ingredion Brasil)), Reisstärke (D.S.A. 7 (Agrana Stärke), Oryzapearl (Ichimaru Pharcos)), Tapiocastärke (Tapioca Pure, Natrasorb W 28-1825, Dry Flo TS (Akzo Nobel)), Distarch Phosphate (Mais PO4 PH"B" (Agrana Stärke)), Corn PO4 (Tri-K)), Kartoffelstärke (Structure Solanace (Akzo Nobel)) , Natriumstärkeoctenylsuccinat (C*EmCap 12633 (Cargill), Cleargum CO 01 (Roquette)) und Calciumstärkeoctenylsuccinat (Dry Flo BF (Akzo Nobel)).

Erfindungsgemäß vorteilhaft enthalten die Wasser-in-Öl-Emulsionen 0,01 bis 20,0 Gew.-%, bevorzugt 0,02 bis 10,0 Gew.-%, besonders bevorzugt 0,1 bis 5,0 Gew.-%, bezogen auf das Gesamtgewicht der Zubereitung, an einer oder mehreren partikulären und/oder nicht-wasserquellbaren Stärken oder Stärkederivaten.

Erfindungsgemäß vorteilhaft enthalten Wasser-in-Öl-Emulsionen 0,1 bis 10,0 Gew.-%, bevorzugt 0,2 bis 5,0 Gew.-%, besonders bevorzugt 0,5 bis 4,0 Gew.-%, bezogen auf das Gesamtgewicht der Zubereitung, an Cetyl Diglyceryl Tris-(Trimethylsiloxy)silylethyldimethicone.

Es war nicht zu erwarten gewesen, dass die erfindungsgemäßen Zubereitungen
- besser als feuchtigkeitsspendende Zubereitungen wirken,
- einfacher zu formulieren sein,
- besser die Hautglättung fördern,
- sich durch besser Pflegewirkung auszeichnen,
- besser als Vehikel für kosmetische und dermatologische Wirkstoffe dienen
- bessere sensorische Eigenschaften, wie beispielsweise die Verteilbarkeit auf der Haut oder das Einzugsvermögen in die Haut, aufweisen würden
- besser als Vehikel für organische UV-Filter geeignet sind
- höhere Stabilität gegenüber Zerfall in Öl- und Wasserphasen aufweisen und
- sich durch bessere Bioverträglichkeit auszeichnen würden
- die Herstellung von stabilen, insbesondere dünnflüssigen Formulierungen ermöglichen als die Zubereitungen des Standes der Technik.

Die erfindungsgemäßen Zubereitungen sind fließfähig aber auch cremeartig formulierbar, besitzen sehr gute kosmetische Eigenschaften, insbesondere was die Klebrigkeit betrifft, und weisen sehr gute Hautverträglichkeit sowie Hautpflegeleistung auf.

Erfindungsgemäß ist es möglich und vorteilhaft, den Anteil der Ölphase der erfindungsgemäßen Zubereitungen im Bereich von 3 bis 60 Gew.-%, bezogen auf das Gesamtgewicht der Zubereitungen, frei zu wählen.

Die erfindungsgemäße Öl- bzw. Lipidphase kann dabei alle in kosmetischen Zubereitungen üblichen Öle, Fette, Wachse und/oder Lipide enthalten.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Ethylenglykol, Ethylenglykolmonoethyl- oder - monobutylether, Propylenglykolmonomethyl, Butyleneglykol, -monoethyl- oder -monobutylether, Diethylenglykolmonomethyl- oder -monoethylether und analoge Produkte, Phenoxyethanol, Methylparaben, Schaumstabilisatoren, Elektrolyte, Selbstbräuner etc.

Als Grundbestandteile der erfindungsgemäßen Zubereitungen können verwendet werden:
- Wasser oder wässrige Lösungen
- wässrige ethanolische Lösungen
- natürliche Öle und/oder chemisch modifizierte natürliche Öle und/oder synthetische Öle;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet.

Die Ölphase der Emulsionen im Sinne der vorliegenden Erfindung besteht erfindungsgemäß wenigstens zum Teil aus Silikonölen.

Es wird bevorzugt, die Ölphase der erfindungsgemäßen Zubereitungen aus der Gruppe der cyclischen und/oder linearen Silicone zu wählen, welche im Rahmen der vorliegenden Offenbarung auch als "Silikonöle" bezeichnet werden. Solche Silicone oder Silikonöle können als Monomere vorliegen, welche in der Regel durch Strukturelemente charakterisiert sind, wie folgt:

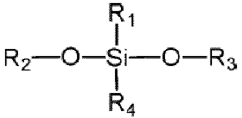

Als erfindungsgemäß vorteilhaft einzusetzende lineare Silicone mit mehreren Siloxyleinheiten werden im Allgemeinen durch Strukturelemente charakterisiert wie folgt: 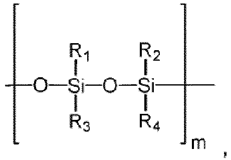 wobei die Siliciumatome mit gleichen oder unterschiedlichen Alkylresten und/oder Arylresten substituiert werden können, welche hier verallgemeinernd durch die Reste R1-R4 dargestellt sind (will sagen, dass die Anzahl der unterschiedlichen Reste nicht notwendig auf bis zu 4 beschränkt ist). m kann dabei Werte von 2 bis 200.000 annehmen.

Vorteilhaft wird Dimethicone als Siliconöl gewählt. Auch andere Silikonöle, beispielsweise Cyclomethicon (Octamethylcyclotetrasiloxan), Phenyldimethicon, beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Es ist allerdings möglich, ohne große Nachteile in Kauf zu nehmen, Ölkomponente aus der Gruppe anderen Ölkomponente zu wählen.

Indessen ist es bevorzugt, die Ölkomponenten der Ölphase der erfindungsgemäßen Zubereitungen vollständig oder weitestgehend vollständig aus der Gruppe der Öle synthetischen und/oder natürlichen Ursprungs zu wählen. Diese können dann vorteilhaft gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, n-Decyloleat, Isooctylstearat, Isononylstearat, Isononylisononanoat, Cetearylisononanoat, Isodecylneopentanoate, 2-Ethylhexylpalmitat, 2-Ethylhexyllaurat, 2-Hexyldecylstearat, Coco-Caprylat/Caprat, Oleyloleat, Oleylerucat, Erucyloleat, Erucylerucat sowie synthetische, halbsynthetische und natürliche Gemische solcher Ester, z.B. Jojobaöl.

Ferner kann die Ölphase teilweise vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Caprylic Capric Triglycerid, Cocoglycerides, Sonnenblumenöl, Sojaöl, Mandelöl und dergleichen mehr.

Vorteilhaft sind ferner Mischungen aus Dimethicon und Cetearylisononanoate.

Von den Kohlenwasserstoffen sind Paraffinöl, Squalan, hydriertes Polyisobuten, Isohexadecan, Isododecan; C₁₅₋₁₉ Alkan bzw. Polydecen vorteilhaft im Sinne der vorliegenden Erfindung zu verwenden.

Zubereitungen entsprechend der vorliegenden Erfindung können auch einen oder mehrere zusätzliche Nicht-Silikon-Emulgatoren enthalten, beispielsweise vorteilhaft gewählt aus der Gruppe der folgenden Substanzen, die in der Regel als W/O-Emulgatoren wirken.

Sorbitanstearat, Sorbitanoleat, Glycerylisostearat, Polyglyceryl-3-Oleat, Wollwachssäuregemische, Wollwachsalkoholgemische, Polyglyceryl-3 Diisostearat, Methylglucosedioleat, Polyglyceryl-4-isostearat, Cetyldimethiconcopolyol, Sorbitanisostearat, Polyglyceryl-2-dipolyhyd-roxystearat, Polyglyceryl-3-Diisostearat, Polyglyceryl-4-dipolyhydroxystearat, PEG-30-dipoly-hydroxystearat, Diisostearoylpolyglyceryl-3-diisostearat, Polyglyceryl-2-dipolyhydroxystearat, Polyglyceryl-3-dipolyhydroxystearat, Polyglyceryl-4-dipolyhydroxystearat.

Erfindungsgemäße Emulsionen im Sinne der vorliegenden Erfindung, z.B. in Form einer Hautschutzcrème, einer Hautlotion, einer kosmetischen Milch, beispielsweise in Form einer Sonnenschutzcreme oder einer Sonnenschutzmilch, sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist dem Fachmanne natürlich bekannt, dass anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, Pigments, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelte entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Günstig sind auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- Ester der Zimtsäure, vorzugsweise 4-Methoxyzimtsäure(2-ethylhexyl)ester
- Ester der Salicylsäure, vorzugsweise Salicylsäure(2-ethylhexyl)ester, Salicylsäure(4-isopropylbenzyl)ester,
- Derivate des Benzophenons, vorzugsweise 2-Hydroxy-4-methoxybenzophenon, 2-Hyd-roxy-4-methoxy-4'-methylbenzophenon, 2,2'-Dihydroxy-4-methoxybenzophenon;
- 2,4,6-Trianilino-(p-carbo-2'-ethyl-1'-hexyloxy)-1,3,5-triazin

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzo-phenon-5-sulfonsäure und ihre Salze;
- Sulfonsäure-Derivate des 3-Benzylidencamphers, wie z.B. 4-(2-Oxo-3-bornylidenme-thyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Chroms, Cers und Mischungen davon, sowie Abwandlungen, bei denen die Oxide die aktiven Agentien sind. Aber auch Ultramarinblau und Perlglanzpigmente. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, organische Lösungsmittel oder Silikonderivate.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Zur Herstellung der erfindungsgemäßen Emulsionen wird der W/O-Emulgator zur Ölphase zugegeben. Die Komponenten der Ölphase sind zusammen mit einem geeigneten Rührer bis zur Homogenität der Phase vermengt, wobei gegebenenfalls leicht erwärmt wird.

Das Natriumchlorid und die jeweilige Stärke sind in der Wasserphase dispergiert. Danach wird die Wasserphase unter Rühren langsam zur Ölphase hinzugegeben und anschließend mit einem hochtourigen Mixer (z. B. einem Zauberstab ESGE) ca. 2 Minuten intensiv vermischt.

Zur Bestimmung der Lagerstabilität werden alle Emulsionsproben in verschraubten 30 ml Gläsern bei unterschiedlichen Temperaturen gelagert: bei Raumtemperatur (RT) (ca. 20 °C), bei +40 °C und bei +50 °C.

Die Stabilität wurde nach 7 Tagen, 14 Tagen, 21 Tagen (T) und 1 Monat (M) visuell bewertet.

| Beispielrezeptur/ Temperaturen | + 40 °C | + 50 °C | RT |
|---|---|---|---|
| 1 - Formelbasis ohne Stärke | Stabil | Leicht instabil ab 7T | Leicht instabil ab 1M |
| 2 - Basis mit **Aluminum Starch Octenylsuccinate** | Leicht instabil ab 7T | Instabil ab 7T | Leicht instabil ab 7T |
| 3 - Basis mit **Nylon-12** *(Klassisches Puderrohstoff*) | Leicht instabil ab 7T | Instabil ab 7T | Leicht instabil ab 1M |
| 4 - Basis mit **Tapioca Stärke** | Stabil | Leicht instabil ab 7T | Stabil |

| | | | |
|---|---|---|---|
| "Stabil" bedeutet, dass keine oder nur geringe Phasentrennung, keine oder nur geringe Ölabscheidung auftritt. "Leicht Instabil" bedeutet, dass es zu einer moderaten, sichtbaren Phasentrennung oder deutlich sichtbaren Ölabscheidung kommt. "Instabil" bedeutet schnelle, vollständige Phasentrennung. | | | |

Diese Experimente haben gezeigt, dass die W/O Emulsionen mit ES-5600 von Dow Corning durch die erfindungsgemäßen Stärken im Vergleich zu anderen etablierten Sensorikadditiven wie Nylon oder Aluminiumstarchoctenylsuccinate am besten stabilisiert bzw weniger destabilisiert wurden.

Die folgenden Beispiele sollen die Erfindung näher erläutern. Wenn nicht anders angegeben, bedeuten die Angaben Gewichts-%. Nur die Beispielrezepturen enthaltend Tapiocastärke sind erfindungsgemäss.

| **Beispielrezepturen / Gew.-%** | 1 | 2 | 3 | 4 |
|---|---|---|---|---|
| **Aluminum Starch Octenylsuccinate** | | 4 | | |
| **Nylon-12** | | | 4 | |
| **Tapioca Stärke** | | | | 4 |
| Cetyl Diglyceryl Tris-(Trimethylsiloxy)silyle-thyldimethicone ("ES-5600") | 1 | 1 | 1 | 1 |
| Cetearylisononanoate | 5 | 5 | 5 | 5 |
| Dimethicon | 15 | 15 | 15 | 15 |
| Glycerin | 7 | 7 | 7 | 7 |
| Natriumchlorid | 0,5 | 0,5 | 0,5 | 0,5 |
| Wasser | Ad 100 | Ad 100 | Ad 100 | Ad 100 |

| **Beispielrezeptur 5** | **Gew.-%** |
|---|---|
| Distärkephosphate | **4** |
| ES-5600 | 1 |
| Cetearylisononanoate | 5 |
| Dimethicon | 15 |
| Glycerin | 7 |
| Natriumchlorid | 0,5 |
| Wasser | Ad 100 |

| **Beispielrezeptur 6** | **Gew.-%** |
|---|---|
| Cetyl Diglyceryl Tris-(Trimethylsiloxy)silylethyldimethicone (ES-5600) | 1 |
| Natriumstärkeoctenylsuccinate | 4 |
| Isononylisononanoate | 15 |
| Dimethicone | 5 |
| Glycerin | 5 |
| Natriumchlorid | 1 |
| Wasser | Ad 100 |

| **Beispielrezeptur 7** | **Gew.-%** |
|---|---|
| Cetyl Diglyceryl Tris-(Trimethylsiloxy)silylethyldimethicone (ES-5600) | 2 |
| Distärkephosphate | 2 |
| Caprylic/Capric Triglyceride | 3 |
| Cocoglycerides | 2 |
| Dimethicone | 10 |
| Disteardimonium Hektorit | 0,2 |
| Propylene Carbonat | 0,05 |
| Butylmethoxydibenzoylmethan | 1 |
| Octocrylen | 2 |
| Octylsalicylat | 2 |
| Natriumascorbylphosphate | 0.1 |
| Ubiquinon | 0.1 |
| Butylenglycol | 2 |
| Tocopherolacetat | 0.1 |
| Farbpigmente (CI 77891, CI 77492, CI 77491, CI 77499, CI 77007) | 6 |
| Panthenol | 0.5 |
| Phenoxyethanol | 0.3 |
| EDTA | 1 |
| Glycerin | 5 |
| Natriumchlorid | 2 |
| Wasser | Ad 100 |

| **Beispielrezepturen 8** | **Gew.-%** |
|---|---|
| Cetyl Diglyceryl Tris-(Trimethylsiloxy)silylethyldimethicone (ES-5600) | 2 |
| Tapicoa Stärke | 2 |
| Caprylic/Capric Triglyceride | 2 |
| C12-15 Alkylbenzoat | 2 |
| Isohexadecan | 2 |
| Dimethicone | 5 |
| Cetearylisononanoate | 2 |
| Bis-Ethylhexyloxyphenol Methoxyphenyl Triazin | 1 |
| Octocrylen | 2 |
| Ethylhexylmethoxyzinnamat | 2 |
| Phenylbenzimidazolsulfonsäure | 1 |
| Kreatin | 0.2 |
| Octandiol | 0.3 |
| Methylpropandiol | 2 |
| Phenoxyethanol | 0.3 |
| EDTA | 1 |
| Glycerin | 5 |
| Natriumchlorid | 2 |
| Wasser | Ad 100 |

## Patentansprüche

1. Kosmetische oder dermatologische Wasser-in-Öl- Emulsionen, enthaltend
A) Cetyl Diglyceryl Tris-(Trimethylsiloxy)silylethyldimethicone als Emulgator
B) eine Ölphase
C) eine wässrige Phase,
**gekennzeichnet durch** einen Gehalt an einer oder mehreren partikulären und/oder nichtwasserquellbaren Stärken und/oder Stärkederivaten, gewählt aus der Gruppe Tapiocastärke, wobei eine Rezeptur wie folgt:
| | Gew.-% |
|---|---|
| ES-5600 (Cetyl Diglyceryl Tris-(Trimethylsiloxy)silylethyldimethicone) | 1,5 |
| Cetearylisononanoate | 8 |
| Dimethicone | 5 |
| Silica | 3 |
| Tapiocastärke | 4 |
| Natriumchlorid | 1 |
| Glycerin | 9 |
| Phenoxyethanol | 0,8 |
| Mineralische Pigmente [Silica + (CI 77491, CI 77891)] - Ronaflair^{®} Flawless | 2 |
| Farbpigmente (CI 77891, CI 77492, CI 77491, CI 77499, CI 77007) | 5 |
| Wasser | Ad 100 |
vom Patentschutz ausgenommen ist.

2. Emulsionen nach Anspruch 1, **dadurch gekennzeichnet, dass** sie 0,1 bis 10,0 Gew.-%, bevorzugt 0,2 bis 5,0 Gew.-%, besonders bevorzugt 0,5 bis 4,0 Gew.-%, bezogen auf das Gesamtgewicht der Zubereitung, an Cetyl Diglyceryl Tris-(Trimethylsiloxy)silylethyldimethicone enthalten.

3. Emulsionen nach einem der vorstehenden Ansprüche, **dadurch gekennzeichnet, dass** sie 0,01 bis 20,0 Gew.-%, bevorzugt 0,02 bis 10,0 Gew.-%, besonders bevorzugt 0,1 bis 5,0 Gew.-%, bezogen auf das Gesamtgewicht der Zubereitung, an einer oder mehreren partikulären und/oder nicht-wasserquellbaren Stärken und/oder Stärkederivaten enthalten.

## Claims

1. Cosmetic or dermatological water-in-oil emulsions, comprising
A) Cetyl Diglyceryl Tris(Trimethylsiloxy)silylethyl Dimethicone as emulsifier,
B) an oil phase,
C) an aqueous phase,
**characterized by** a content of one or more particulate and/or non-water-swellable starches and/or starch derivatives, selected from the group of tapioca starch, where a formulation as follows:
| | % by weight |
|---|---|
| ES-5600 (Cetyl Diglyceryl Tris(Trimethylsiloxy)silylethyl Dimethicone) | 1.5 |
| Cetearyl isononanoate | 8 |
| Dimethicone | 5 |
| Silica | 3 |
| Tapioca starch | 4 |
| Sodium chloride | 1 |
| Glycerin | 9 |
| Phenoxyethanol | 0.8 |
| Mineral pigments [Silica + (CI 77491, CI 77891)] - Ronaflair^{®} Flawless | 2 |
| Colour pigments (CI 77891, CI 77492, CI 77491, CI 77499, CI 77007) | 5 |
| Water | to 100 |
is excluded from patent protection.

2. Emulsions according to Claim 1, **characterized in that** they comprise 0.1% to 10.0% by weight, preferably 0.2% to 5.0% by weight, particularly preferably 0.5% to 4.0% by weight, based on the total weight of the preparation, of Cetyl Diglyceryl Tris(Trimethylsiloxy)silylethyl Dimethicone.

3. Emulsions according to either of the preceding claims, **characterized in that** they comprise 0.01% to 20.0% by weight, preferably 0.02% to 10.0% by weight, particularly preferably 0.1% to 5.0% by weight, based on the total weight of the preparation, of one or more particulate and/or non-water-swellable starches and/or starch derivatives.

## Revendications

1. Emulsions cosmétiques ou dermatologiques eau-dans-huile, contenant
A) de la cétyl diglycéryl tris-(triméthylsiloxy)silyléthyldiméthicone en tant qu'émulsifiant,
B) une phase huileuse,
C) une phase aqueuse,
**caractérisées par** une teneur en un ou plusieurs amidons et/ou dérivés d'amidon particulaires et/ou non gonflables à l'eau, choisis dans le groupe de l'amidon de tapioca, une formulation telle que suit :
| | % en poids |
|---|---|
| ES-5600 (cétyl diglycéryl tris-(triméthylsiloxy)silyléthyldiméthicone) | 1,5 |
| Cétéarylisononanoate | 8 |
| Diméthicone | 5 |
| Silice | 3 |
| Amidon de tapioca | 4 |
| Chlorure de sodium | 1 |
| Glycérine | 9 |
| Phénoxyéthanol | 0,8 |
| Pigments minéraux [silice + (CI 77491, CI 77891)] - Ronaflair^{®} Flawless | 2 |
| Pigments colorants (CI 77891, CI 77492, CI 77491, CI 77499, CI 77007) | 5 |
| Eau | Jusqu'à 100 |
étant exclue de la protection par brevet.

2. Émulsions selon la revendication 1, **caractérisées en ce qu'**elles contiennent 0,1 à 10,0 % en poids, de préférence 0,2 à 5,0 % en poids, de manière particulièrement préférée 0,5 à 4,0 % en poids, par rapport au poids total de la préparation, de cétyl diglycéryl tris-(triméthylsiloxy)silylethyldiméthicone.

3. Émulsions selon l'une des revendications précédentes, **caractérisées en ce qu'**elles contiennent 0,01 à 20,0 % en poids, de préférence 0,02 à 10,0 % en poids, de manière particulièrement préférée 0,1 à 5,0 % en poids, par rapport au poids total de la préparation, d'un ou plusieurs amidons et/ou dérivés d'amidon particulaires et/ou non gonflables à l'eau.
